(19) Europäisches Patentamt
European Patent Office
Office européen des brevets

(11) Numéro de publication: **0 196 260 B1**

(12) **FASCICULE DE BREVET EUROPEEN**

(45) Date de publication de fascicule du brevet: **21.08.91**

(51) Int. Cl.[5]: **C07C 37/62**, C07C 37/86, C07C 41/22, C07C 43/23

(21) Numéro de dépôt: **86420056.3**

(22) Date de dépôt: **20.02.86**

Le dossier contient des informations techniques présentées postérieurement au dépôt de la demande et ne figurant pas dans le présent fascicule.

# (54) Procédé de chloration sélective de composés phénoliques.

(30) Priorité: **12.03.85 FR 8503802**

(43) Date de publication de la demande: **01.10.86 Bulletin 86/40**

(45) Mention de la délivrance du brevet: **21.08.91 Bulletin 91/34**

(84) Etats contractants désignés: **AT BE CH DE FR GB IT LI LU NL SE**

(56) Documents cités:
**DE-A- 3 318 791**
**GB-A- 2 135 310**

**CHEMICAL ABSTRACTS, vol. 93, no. 25, 22 décembre 1980, page 814, no. 239023s, Columbus, Ohio, US; & JP - A - 80 40 613**

**Jerry March, Advanced Organic Chemistry, p. 246-248**

(73) Titulaire: **RHONE-POULENC CHIMIE**
**25, quai Paul Doumer**
**F-92408 Courbevoie Cédex(FR)**

(72) Inventeur: **Ratton, Serge**
**Hameau de Belmont Vaulx Milieu**
**F-38090 Villefontaine(FR)**
Inventeur: **Leblanc, Jean-Claude**
**10, boulevard Joffre**
**F-38000 Grenoble(FR)**

(74) Mandataire: **Vignally, Noel et al**
**RHONE-POULENC INTERSERVICES Service Brevets Chimie Centre de Recherches de Saint-Fons B.P. 62**
**F-69192 Saint-Fons Cedex(FR)**

**EP 0 196 260 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne la chloration sélective en position ortho par rapport à la fonction hydroxyle de certains composés phénoliques en milieu solvant, par le chlore gazeux.

Dans la préparation des chlorophénols par chloration du phénol, on obtient, aux divers stades de la réaction, des isomères, ce qui conduit finalement à de nombreux composés, qu'il est ensuite nécessaire de séparer par des opérations coûteuses et délicates.

Ainsi l'action du chlore sur le phénol en milieu fondu conduit à un mélange des isomères chloro-2 phénol et chloro-4 phénol (environ 35 % de chloro-2 phénol et 65 % de chloro-4 pnénol).

Il est donc interessant de pouvoir disposer pour chaque stade de la chloration du phénol ou des chlorophénols, de procédés sélectifs conduisant exclusivement ou très majoritairement à l'un des isomères. C'est ainsi que la demande de brevet allemand n° 3 318 791 décrit un procédé sélectif de chloration du phénol en chloro-2 phénol, dans un solvant non polaire perchloré et en présence d'une amine à chaîne ramifiée.

Ce document indique à la page 19 que, pour des concentrations en amine supérieures à 0,0500 % par rapport au solvant, l'orthochlorophénol formé tend à se chlorer en dichloro-2,6 phénol.

Le brevet JP-A-80.40 613 décrit la chloration des alkyl-4 phénols en dichloro-2,6 alkyl-4 phénols par le chlore, dans un solvant chloré et en présence d'une amine.

En poursuivant les recherches dans ce domaine, il a été trouvé que l'on peut également chlorer sélectivement en position ortho par rapport à la fonction OH, certains autres composés phénoliques qui comportent des substituants ayant un effet inductif inverse de celui des groupements alkyle, comme on peut le voir dans "Advanced Organic Chemistry" de Jerry MARCH, tableau 5 page 247 (Mc GRAW-HILL BOOK COMPANY, Edition de 1968).

Plus précisément la présente invention est un procédé de chloration sélective en position ortho par rapport à la fonction OH des composés phénoliques de formule générale (I) :

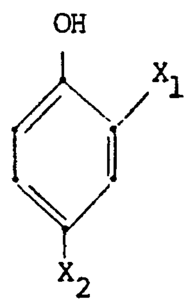

(I)

dans laquelle :

- $X_1$ représente un atome de chlore, un groupement OH, un groupement alkyle ayant 1 à 4 atomes de carbone, un groupement alkoxy ayant 1 à 4 atomes de carbone, un groupement aldéhyde ou un atome d'hydrogène,
- $X_2$ représente un atome de chlore, un groupement OH, un groupement alkoxy ayant 1 à 4 atomes de carbone, un groupement aldéhyde ou un atome d'hydrogène,
- les symboles $X_1$ et $X_2$ ne représentent pas simultanément un atome d'hydrogène,
- le symbole $X_1$ ne représentant pas un atome de chlore lorsque $X_2$ représent un atome d'hydrogène,

par le chlore gazeux, caractérisé en ce que l'on opère dans un solvant aprotique apolaire et en présence d'une quantité efficace d'une amine de formule générale (II) :

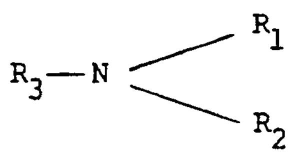

(II)

dans laquelle :

- $R_1$, $R_2$ et $R_3$ identiques ou différents représentent un radical alkyle linéaire ayant de 1 à 10 atomes de carbone, un radical alkyle secondaire ayant de 3 à 10 atomes de carbone ou un radical alkyle tertiaire ayant de 4 à 10 atomes de carbone,

- $R_1$, $R_2$ ou $R_3$ représente un radical phényle, un radical cyclohexyle, un radical cycloheptyle ou un radical cyclopentyle,
- un ou deux des symboles $R_1$, $R_2$, $R_3$ peuvent représenter un atome d'hydrogène.

L'invention concerne également un procédé de chloration sélective en position ortho par rapport à la fonction OH du chloro-2 phénol exempt de phénol, par le chlore gazeux, caractérisé en ce que l'on opère dans un solvant aprotique apolaire et en présence de 0,0010 % à 0,0200 % en poids d'une amine ramifiée de formule générale (II), par rapport au solvant aprotique apolaire :

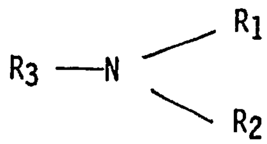

(II)

dans laquelle :
- $R_1$, $R_2$ et $R_3$ identiques ou différents représentent un radical alkyle linéaire ayant de 1 à 10 atomes de carbone, un radical alkyle secondaire ayant de 3 à 10 atomes de carbone ou un radical alkyle. tertiaire ayant de 4 à 10 atomes de carbone,
- $R_1$, $R_2$ ou $R_3$ représente un radical phényle, un radical cyclohexyle, un radical cycloheptyle ou un radical cyclopentyle,
- un ou deux des symboles $R_1$, $R_2$ et $R_3$ peuvent représenter un atome d'hydrogène.

Parmi les amines de formule (II) on peut citer à titre d'exemples illustratifs :
- des amines primaires telles que la n-butylamine, la n-pentylamine, la n-hexylamine, l'aniline, la cyclohexylamine ;
- des amines secondaires telles que la dibutylamine, la dipropylamine ;
- des amines tertiaires telles que la tributylamine, la triéthylamine.

Parmi les amines de formule (II) on préférera plus particulièrement les amines à chaîne ramifiée de formule générale (III):

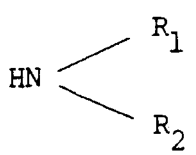

(III)

dans laquelle :
- $R_1$ représente un radical alkyle secondaire ayant de 3 à 10 atomes de carbone ou un radical alkyle tertiaire ayant de 4 à 10 atomes de carbone,
- $R_2$ représente :
  + un atome d'hydrogène lorsque $R_1$ est un radical alkyle tertiaire,
  + un radical alkyle secondaire ayant de 3 à 10 atomes de carbone lorsque $R_1$ est également un radical alkyle secondaire.

Les solvants aprotiques apolaires utilisés dans le procédé selon l'invention sont essentiellement les hydrocarbures aliphatiques, les hydrocarbures aliphatiques chlorés et les chlorobenzènes et bromobenzènes.

A titre d'hydrocarbures aliphatiques on peut oiter notamment l'hexane, l'heptane, l'octane, le nonane et le décane.

Parmi les hydrocarbures chlorés on peut citer les hydrocarbures perchlorés et les hydrocarbures partiellement chlorés tels que le chlorure de méthylène, le dichloroéthane, le tétrachloroéthane, le trichloroéthylène, le chloro-1 butane, le dichloro-1,2 butane.

Comme chlorobenzènes on peut utiliser en particulier le monochlorobenzène, le dichloro-1,2 benzène, le dichloro-1,3 benzène le dichloro-1,4 benzène ou des mélanges de différents chlorobenzènes ; comme bromobenzènes on emploie le plus souvent le monobromobenzène ou des mélanges de monobromobenzène avec un ou plusieurs dibromobenzènes.

Les hydrocarbures perchlorés sont particulièrement adaptés au procédé de l'invention ; ce sont de préférence les alcanes perchlorés ayant 1 à 4 atomes de carbone, les alcènes perchlorés ayant 2 à 4 atomes de carbone et les alcadiènes perchlorés ayant 3 à 6 atomes de carbone.

A titre d'exemples de ces hydrocarbures perchlorés on peut citer notamment le tétrachlorure de

carbone, le tétrachloroéthylène, l'hexachloroéthane, l'hexachloropropène et l'hexachlorobutadiène.

Les plus utilisés parmi ces hydrocarbures perchlorés sont le tétrachlorure de carbone et le tétrachloroéthylène.

Parmi les composés phénoliques de formule (I), on utilise plus particulièrement ceux pour lesquels :

- $X_1$ et $X_2$, identiques ou différents, représentent un atome de chlore, un groupement OH, un groupement méthoxy, un groupement éthoxy ou un groupement aldéhyde,
- un seul des symboles $X_1$ ou $X_2$ pouvant représenter un atome d'hydrogène.

Le présent procédé s'applique de préférence au chloro-2 phénol, au chloro-4 phénol, au dichloro-2,4 phénol ainsi qu'aux mélanges de deux d'entre-eux et aux mélanges de l'un ou de deux d'entre-eux avec d'autres composés, notamment avec d'autres chlorophénols.

Il s'applique également de manière intéressante au pyrocatéchol, au gaïacol, au méthoxy-4 phénol, à l'éthoxy-2 phénol, a l'éthoxy-4 phénol, à l'hydroxy-2 benzaldéhyde et à l'hydroxy-4 benzaldéhyde.

La réaction est généralement conduite à une température comprise entre 40 °C et la température à laquelle le milieu réactionnel est sous reflux.

De préférence la température à laquelle est mis en oeuvre le procédé de l'invention est comprise entre 60 °C et 120 °C.

Bien qu'en règle générale une concentration initiale (ou, lorsque l'on opère par introduction en continu du composé phénolique, la concentration instantanée) relativement peu élevée dans le milieu réactionnel favorise la sélectivité de la chloration en position ortho par rapport à la fonction OH, la concentration en composé phénolique de formule (I) ou en mélange de composés phénoliques comprenant un ou deux composés phénoliques de formule (I) dans le milieu réactionnel peut varier largement par exemple entre 1 % et 50 % en poids par rapport au solvant aprotique apolaire utilisé. L'utilisation d'une concentration initiale plus faible, par exemple entre 2 % et 10 % en poids permet d'obtenir une sélectivité de la chloration en position ortho par rapport à la fonction OH plus élevée, mais une concentration plus élevée procure bien évidemment une productivité de l'appareillage supérieure pour une sélectivité encore importante.

Parmi les amines à chaîne ramifiée de formule (III) qui catalysent particulièrement bien l'orientation de chloration des composés phénoliques de formule (I) en position ortho par rapport à la fonction OH on peut citer :

- à titre d'exemple d'amines secondaires
  . celles qui possèdent deux radicaux alkyles secondaires tels que isopropyle, butyle-2, pentyle-2, pentyle-3, hexyle-2, hexyle-3, heptyle-2, heptyle-3, heptyle-4, octyle-2, octyle-3, octyle-4, nonyle-2, nonyle-3, nonyle-4, nonyle-5, décyle-2, décyle-3, décyle-4, décyle-5 ;
- à titre d'exemple d'amines primaires
  . celles qui possèdent un radical alkyle tertiaire ayant de 4 à 10 atomes de carbone tel que tertiobutyle ; diméthyl-1,1 propyle ; diméthyl-1,1 butyle ; diméthyl-1,1 pentyle ; diméthyl-1,1 hexyle ; diméthyl-1,1 heptyle ; diméthyl-1,1 octyle ; méthyl-1 éthyl-1 propyle ; diéthyl-1,1 propyle ; méthyl-1 éthyl-1 butyle ; diéthyl-1,1 butyle ; méthyl-1 propyl-1 butyle ; dipropyl-1,1 butyle ; éthyl-1 propyl-1 butyle ; méthyl-1 éthyl-1 pentyle ; méthyl-1 propyl-1 pentyle ; diéthyl-1,1 pentyle ; éthyl-1 propyl-1 pentyle ; méthyl-1 éthyl-1 hexyle ; méthyl-1 propyl-1 hexyle ; diéthyl-1,1 hexyle ; méthyl-1 éthyl-1 heptyle ;
  . et celles qui comportent un radical alkyle tertiaire dans lequel deux des radicaux liés au carbone lui-même lié à l'atome d'azote, forment ensemble un radical alkylène ayant 2 à 6 atomes de carbone tel que méthyl-1 cyclopentyle, méthyl-1 cyclohexyle ou méthyl-1 cycloheptyle.

Parmi les amines à chaîne ramifiée de formule (III) qui conviennent tout particulièrement bien, on peut citer la diisopropylamine, la tertiobutylamine, la méthyl-1 cyclopentylamine, la méthyl-1 cyclohexylamine.

La quantité d'amine de formule (II) ou (III) que l'on utilise dans le procédé de l'invention * est généralement de 0,0010 % à 0,1000 % en poids par rapport au solvant aprotique apolaire.

De préférence on utilisera de 0,0050 % à 0,0200 % en poids d'amine de formule (II) ou (III) par rapport au solvant aprotique apolaire.

Le présent procédé de chloration des composés phénoliques de formule (I) peut être mis en oeuvre de manière continue ou de manière discontinue.

De manière générale la quantité de chlore engagée est proche de la quantité théorique nécessaire pour chlorer une position ortho des composés phénoliques de formule (I). Ainsi le rapport molaire

* appliqué aux composés de formule (I)

$$\frac{\text{chlore}}{\text{composé phénolique (I)}}$$

est habituellement compris entre 0,9 et 1,1

La durée de réaction est variable, notamment selon la température et le composé phénolique (I) mis en oeuvre.

En règle générale elle se situe entre 30 minutes et quelques heures, le plus fréquemment entre 30 minutes et 2 heures à la température choisie.

Un des objets de l'invention consiste à préparer sélectivement

- du chloro-2,6 phénol à partir du chloro-2 phénol ;
- du dichloro-2,4 phénol à partir du chloro-4 phénol ;
- du trichloro-2,4,6 phénol à partir du dichloro-2,4 phénol.

Une autre application de l'invention consiste à chlorer sélectivement un chlorophénol de formule (I) présent dans un mélange comportant 1 ou plusieurs autres chlorophénols qui ne sont pas affectés par la réaction.

C'est ainsi que l'on peut par exemple purifier du dichloro-2,6 phénol qui se trouve en mélange avec du dichloro-2,4 phénol et éventuellement d'autres chlorophénols. La séparation de ces 2 dichlorophénols est très difficile par les méthodes courantes telles que la distillation. Le procédé de purification du dichloro-2,6 phénol selon la présente invention consistera donc à chlorer sélectivement le dichloro-2,4 phénol contenu dans le dichloro-2,6 phénol, par le chlore gazeux, dans un solvant aprotique apolaire et en présence de 0,0010 % à 0,1000 % en poids par rapport au solvant aprotique apolaire d'une amine de formule générale (II) ou (III) telle que définie précédemment.

Les valeurs des paramètres (température, rapport molaire chlore/dichloro-2,4 phénol), le solvant aprotique apolaire et l'amine de formule (II) ou (III) utilisés sont ceux qui ont été indiqués précédemment. La concentration dans le solvant aprotique apolaire est généralement calculée pour l'ensemble du mélange de chlorophénols et non pour le seul dichloro-2,4 phénol.

La chloration sélective du dichloro-2,4 phénol en trichloro-2,4,6 phénol dans les mélanges comportant du dichloro-2,6 phénol, permet une séparation ultérieure beaucoup plus aisée du dichloro-2,6 phénol par exemple par distillation.

On peut préparer le trichloro-2,4,6 phénol très pur, exempt en particulier de trichloro-2,4,5 phénol à partir d'un mélange de di- et trichlorophénols, en enchaînant des opérations de séparation par distillation et des réactions de chloration. Ainsi à partir d'un mélange principalement constitué de dichloro-2,6 phénol, de dichloro-2,4 phénol, de trichloro-2,4,6 phénol et de divers isomères trichlorophénols et tétrachlorophénols, on effectue une séparation par distillation. On récupère en tête un mélange de dichloro-2,4 phénol et de dichloro-2,6 phénol. On élimine les culots de distillation composés de trichloro-2,4,6 phénol et de divers trichlorophénols et tétrachlorophénols.

On effectue la chloration du mélange dichloro-2,4 phénol et dichloro-2,6 phénol en introduisant une quantité de chlore telle que $Cl_2$/dichloro-2,4 phénol = 1. Ainsi on obtient la transformation quasi totale du dichloro-2,4 phénol en trichloro-2,4,6 phénol, tandis que le dichloro-2,6 phénol ne réagit pas.

Le mélange dichloro-2,6 phénol et trichloro-2,4,6 phénol est rectifié. En tête on recueille le dichloro-2,6 phénol, puis on récupère le trichloro-2,4,6 phénol très pur.

On peut également chlorer sélectivement le chloro-4 phénol en mélange avec le chloro-2 phénol, ce qui permet ensuite la séparation plus facile du chloro-2 phénol non transformé avec le dichloro-2,4 phénol obtenu sélectivement.

Le procédé de purification du chloro-2 phénol selon la présente invention consistera donc à chlorer sélectivement le chloro-4 phénol contenu dans le chloro-2 phénol, par le chlore gazeux, dans un solvant aprotique apolaire et en présence de 0,0010 % à 0,1000 % en poids par rapport au solvant d'une amine de formule générale (II) ou (III) telle que définie précédemment. Les valeurs des paramètres (température, rapport molaire chlore/chloro-4 phénol), le solvant aprotique apolaire et l'amine de formule (II) ou (III) utilisés sont ceux qui ont été indiqués précédemment. La concentration dans le solvant aprotique apolaire est généralement calculée pour l'ensemble du mélange du chlorophénols et non pour le seul chloro-4 phénol.

La chloration sélective en ortho du groupement OH du chloro-2 phénol, du chloro-4 phénol et du dichloro-2,4 phénol permet d'obtenir des composés (dichloro-2,6 phénol, dichloro-2,4 phénol et trichloro-2,4,6 phénol) qui sont des intermédiaires chimiques intéressants utilisés pour la préparation de produits phytosanitaires et de colorants.

En outre la chloration sélective d'un chlorophénol en mélange avec d'autres chlorophénols permet la séparation d'isomères difficiles à isoler directement par les méthodes usuelles telles que la distillation

fractionnée.

Le procédé selon la présente invention permet également, comme indiqué précédemment, de chlorer sélectivement en position ortho des composés phénoliques non chlorés tels que notamment le gaïacol, le méthoxy-4 phénol, l'éthoxy-2 phénol, l'éthoxy-4 phénol, le pyrocatéchol, méthoxy-4 phénol, l'éthoxy-2 phénol, l'éthoxy-4 phénol, le pyrocatéchol, l'hydroxy-2 benzaldéhyde ou l'hydroxy-4 benzaldéhyde. Les exemples qui suivent permettent d'illustrer l'invention.

Exemple 1

Dans un réacteur en verre pyrex de 1000 cm3, muni d'une agitation, on charge :
- 25,71 g de chloro-2 phénol (0,2 mole)
- 612,5 g de tétrachloroéthylène,
- 0,06 g de diisopropylamine (0,010 % en poids par rapport au tétrachloroéthylène).

On chauffe le mélange réactionnel à 110° C et on introduit à l'aide d'un tube plongeur 0,2 mole de chlore gazeux en 55 minutes.

La composition molaire finale est déterminée par analyse chromatographique ; on trouve :
- chloro-2 phénol non transformé : 1,3 % soit un taux de transformation (TT) de 98,7 %
- dichloro-2,6 phénol : 92,2 %, soit un rendement par rapport au chloro-2 phénol transformé (RT) de 94 %.
- dichloro-2,4 phénol : 4,02 %, soit un RT de 4,1 %
- trichloro-2,4,6 phénol : 2,46 %, soit un RT de 2,5 %.

On ne décèle pas de trichloro-2,4,5 phénol (moins de 0,0005 % dans le mélange de chlorophénols).

Le rapport molaire :

$$\frac{\text{dichloro-2,6 phénol formé}}{\text{dichloro-2,4 phénol + trichloro-2,4,6 phénol formés}}$$

est de 14,2 Un essai témoin effectué avec les mêmes réactifs et dans les mêmes conditions, mais en l'absence de diisopropylamine donne un rapport molaire final

$$\frac{\text{dichloro-2,6 phénol}}{\text{dichloro-2,4 phénol + trichloro-2,4,6 phénol}}$$

voisin de 10.

Un autre essai effectué avec les mêmes réactifs, mais en l'absence de solvant et de diisopropylamine donne un rapport molaire final

$$\frac{\text{dichloro-2,6 phénol}}{\text{dichloro-2,4 phénol + trichloro-2,4,6 phénol}}$$

de 0,25 environ.

Exemple 2

Dans un réacteur en verre pyrex de 1000 cm3, muni d'une agitation, on charge :
- 32,60 g de dichloro-2,4 phénol (0,2 mole)
- 612,5 g de tétrachloroéthylène,
- 0,06 g de diisopropylamine (0,010 % en poids par rapport au tétrachloroéthylène).

On chauffe le mélange réactionnel à 110° C et on introduit en 1 heure à l'aide d'un tube plongeur 0,22 mole de chlore gazeux.

On obtient les résultats suivants :

TT du dichloro-2,4 phénol : 99,6 %

RT en trichloro-2,4,6 phénol : 100 %

Trichloro-2,4,5 phénol : inférieur à la limite de détection (moins de 0,0005 % par rapport au trichloro-2,4,6 phénol).

Exemple 3

Dans un réacteur en verre pyrex de 1000 cm3 muni d'une agitation, on charge :

- 8,15 g de dichloro-2,6 phénol (0,05 mole),
- 11,41 g de dichloro-2,4 phénol (0,07 mole),
- 15,8 g de trichloro-2,4,6 phénol (0,08 mole),
- 612,5 g de tétrachloroéthylène,
- 0,06 g de diisopropylamine (0,010 % par rapport au tétrachloroéthylène).

On chauffe le mélange à 110 °C et on introduit en 1 heure à l'aide d'un tube plongeur 0,07 mole de chlore gazeux.

La masse réactionnelle finale est dosée par chromatographie. On trouve :

TT du dichloro-2,4 phénol : environ 100 %

RT en trichloro-2,4,6 phénol par rapport au dichloro-2,4 phénol : 100 % environ

TT du dichloro-2,6 phénol : 2 %

Un essai comparatif effectué en l'absence de tétrachloroéthylène et d'amine conduit aux résultats suivants :

TT du dichloro-2,4 phénol : 45 %

RT en trichloro-2,4,6 phénol par rapport au dichloro-2,4 phénol transformé : 100 % environ

TT du dichloro-2,6 phénol : 37 %

RT en trichloro-2,4,6 phénol par rapport au dichloro-2,6 phénol transformé : 100 % environ

Présence de trichloro-2,4,5 phénol.

Exemples 4 à 9

Une série de chlorations du chloro-2 phénol sont effectuées dans l'appareillage décrit dans l'exemple 1 et selon le même mode opératoire, mais selon les essais :

- avec un solvant aprotique apolaire différent,
- avec une amine différente,
- avec une concentration initiale en chloro-2 phénol différente.

Le tableau ci-après indique les caractéristiques principales des différents exemples (solvant, amine, concentration initiale en chloro-2 phénol, température, durée) ainsi que les résultats obtenus (TT du chloro-2 phénol, RT en dichloro-2,6 phénol, en dichloro-2,4 phénol et en trichloro-2,4,6 phénol) et sélectivité de la chloration (c'est à dire : rapport molaire

$$\frac{\text{dichloro-2,6 phénol obtenu}}{\text{dichloro-2,4 + trichloro-2,4,6 phénols obtenus}} \quad )$$

| Exemples | Solvant aprotique apolaire | Amine (% en poids par rapport au solvant) | concentration initiale en chloro-2 phénol | Température | Durée | TT % du chloro-2 phénol | RT % en dichloro-2,6 phénol | RT % en dichloro-2,4 phénol | RT % en trichloro-2,4,6 phénol * | sélectivité en dichloro-2,6 phénol |
|---|---|---|---|---|---|---|---|---|---|---|
| Exemple 4 | tétrachloro-éthylène | Diisopropyl-amine (0,010 %) | 21,0 | 110°C | 1 h. | 92,6 | 88,9 | 6,3 | 2,2 | 10,4 |
| Exemple 5 | tétrachloro-éthylène | Dibutyl-amine (0,010 %) | 4,2 | 110°C | 1 h. | 100 | 91,3 | 2,3 | 6,4 | 10,5 |
| Exemple 6 | tétrachlo-rure de carbone | Diisopropyl-amine (0,010 %) | 4,2 | 72°C | 1 h. | 97,9 | 96,7 | 2,45 | 0,80 | 29,5 |
| Exemple 7 | Dichloro-1,2 éthane | Diisopropyl-amine (0,010 %) | 4,2 | 67°C | 1 h. | 85,4 | 94,3 | 5,0 | 0,50 | 17,2 |
| Exemple 8 | hexane | Diisopropyl-amine (0,010 %) | 4,2 | 64°C | 1 h. | 90,2 | 95,1 | 4,1 | 0,80 | 19,0 |

* On ne décèle dans aucun des exemples de présence de trichloro-2,4,5 phénol (limite de détection 0,0005 % dans le mélange de chlorophénols).

Exemple 10

Dans un réacteur en verre pyrex de 1000 cm3 muni d'une agitation, on charge :

- 24,83 g de gaïacol (0,2 mole),

- 612,5 g de tétrachloroéthylène,
- 0,06 g de diisopropylamine (0,010 % par rapport au tétrachloroéthylène).

On chauffe le mélange à 110° C et on introduit en 1 heure à l'aide d'un tube plongeur 0,2 mole de chlore gazeux avec un débit de 5 litres/heure pendant 54 minutes (soit 4,48 litres).

La masse réactionnelle finale est dosée par chromatographie.

| | |
|---|---|
| - gaïacol | : 1,13 g |
| - chloro-2 méthoxy-6 phénol | : 26,44 g |
| - chloro-4 méthoxy-2 phénol | : 1,87 g |
| - dichloro-2,4 méthoxy-6 phénol | : 1,61 |

On trouve :

Soit :

- TT du gaïacol : 95,5 %
- RT en chloro-2 méthoxy-6 phénol par rapport au gaïacol : 88 %
- RT en chloro-4 méthoxy-2 phénol par rapport au gaïacol : 6,1 %
- RT en dichloro-2,4 méthoxy-6 phénol par rapport au gaïacol : 4,3 %.

Exemple 11

D'un mélange brut de chlorophénols contenant du dichloro-2,4 phénol, du dichloro-2,6 phénol, du trichloro-2,4,6 phénol, divers trichlorophénols isomères et de faibles quantités de tétrachloro-2,3,4,6 phénol, on retire par distillation :

- 25 g de dichloro-2,6 phénol (0,153 mole)
- 33 g de dichloro-2,4 phénol (0,202 mole).

On dissout ce mélange dans 600 g de tétrachloroéthylène. La solution est introduite dans un réacteur en verre pyrex de 1000 cm3 muni d'une agitation. On rajoute 0,06 g de diisopropylamine (0,010 % en poids par rapport au tétrachloroéthylène).

On chauffe le mélange réactionnel à 110° C et on introduit en 1 heure à l'aide d'un tube plongeur 0,22 mole de chlore gazeux.

On obtient les résultats suivants :

TT du dichloro-2,4 phénol : 99 %

RT en trichloro-2,4,6 phénol : 100 %

Trichloro-2,4,5 phénol : inférieur à la limite de détection (moins de 0,0005 % par rapport au trichloro-2,4,6 phénol)

TT du dichloro-2,6 phénol : 1 %.

Après élimination du tétrachloroéthylène sous pression réduite (environ 6600 pascals) on procède à une distillation fractionnée du mélange du chlorophénols sous environ 660 pascals.

On obtient :

- En tête : dichloro-2,6 phénol : 24 grammes
- dichloro-2,4 phénol : traces
- Une fraction intermédiaire : 2,8 grammes (dichloro-2,6 phénol, traces de dichloro-2,4 phénol et essentiellement du trichloro-2,4,6 phénol)
- Une fraction de coeur : trichloro-2,4,6 phénol : 34 grammes
- Résidu : 6,05 grammes.

## Revendications

**1.** Procédé de chloration sélective en position ortho par rapport à la fonction OH des composés phénoliques de formule générale (I).

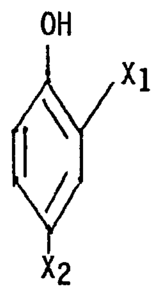

(I)

dans laquelle :

- $X_1$ représente un atome de chlore, un groupement OH, un groupement alkyle ayant 1 à 4 atomes de carbone, un groupement alkoxy ayant 1 à 4 atomes de carbone, un groupement aldéhyde ou un atome d'hydrogène,
- $X_2$ représente un atome de chlore, un groupement OH, un groupement alkoxy ayant 1 à 4 atomes de carbone, un groupement aldéhyde ou un atome d'hydrogène,
- les symboles $X_1$ et $X_2$ ne représentent pas simultanément un atome d'hydrogène,
- le symbole $X_1$ ne représentant pas un atome de chlore lorsque $X_2$ représente un atome d'hydrogène,

par le chlore gazeux, caractérisé en ce que l'on opère dans un solvant aprotique apolaire et en présence d'une quantité efficace d'une amine de formule générale (II) :

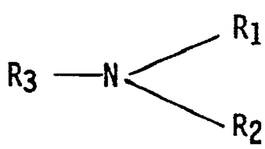

(II)

dans laquelle :

- $R_1$, $R_2$ et $R_3$ identiques ou différents représentent un radical alkyle linéaire ayant de 1 à 10 atomes de carbone, un radical alkyle secondaire ayant de 3 à 10 atomes de carbone ou un radical alkyle tertiaire ayant de 4 à 10 atomes de carbone, $R_1$, $R_2$ ou $R_3$ représente un radical phényle, un radical cyclohexyle, un radical cycloheptyle ou un radical cyclopentyle,
- un ou deux des symboles $R_1$, $R_2$ et $R_3$ peuvent représenter un atome d'hydrogène.

**2.** Procédé de chloration sélective en position ortho par rapport à la fonction OH du chloro-2 phénol exempt de phénol, par le chlore gazeux, caractérisé en ce que l'on opère dans un solvant aprotique apolaire et en présence de 0,0010 % à 0,0200 % en poids d'une amine de formule générale (II), par rapport au solvant aprotique apolaire :

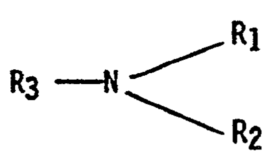

(II)

dans laquelle :

- $R_1$, $R_2$ et $R_3$ identiques ou différents représentent un radical alkyle linéaire ayant de 1 à 10 atomes de carbone, un radical alkyle secondaire ayant de 3 à 10 atomes de carbone ou un radical alkyle tertiaire ayant de 4 à 10 atomes de carbone, $R_1$, $R_2$ ou $R_3$ représente un radical phényle, un radical cyclohexyle, un radical cycloheptyle ou un radical cyclopentyle,
- un ou deux des symboles $R_1$, $R_2$ et $R_3$ peuvent représenter un atome d'hydrogène.

**3.** Procédé selon la revendication 2, caractérisé en ce que l'on utilise une amine à chaîne ramifiée de formule générale (III)

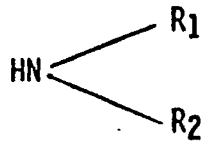

(III)

dans laquelle :

- $R_1$ représente un radical alkyle secondaire ayant de 3 à 10 atomes de carbone ou un radical alkyle tertiaire ayant de 4 à 10 atomes de carbone,
- $R_2$ représente :
  + un atome d'hydrogène lorsque $R_1$ est un radical alkyle tertiaire,
  + un radical alkyle secondaire ayant de 3 à 10 atomes de carbone lorsque $R_1$ est également un radical alkyle secondaire.

**4.** Procédé selon la revendication 1, caractérisé en ce que l'on utilise une amine à chaîne ramifiée de formule générale (III)

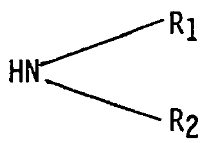

(III)

dans laquelle :

- $R_1$ représente un radical alkyle secondaire ayant de 3 à 10 atomes de carbone ou un radical alkyle tertiaire ayant de 4 à 10 atomes de carbone,
- $R_2$ représente :
  + un atome d'hydrogène lorsque $R_1$ est un radical alkyle tertiaire,
  + un radical alkyle secondaire ayant de 3 à 10 atomes de carbone lorsque $R_1$ est également un radical alkyle secondaire.

**5.** Procédé selon l'une des revendications 1 ou 4, caractérisé en ce qu'il est appliqué aux composés phénoliques de formule (I), dans laquelle :

- $X_1$ et $X_2$ identiques ou différents, représentent un atome de chlore, un groupement OH, un groupement méthoxy, un groupement éthoxy ou un groupement aldéhyde,
- un seul des symboles $X_1$ OU $X_2$ pouvant représenter un atome d'hydrogène.

**6.** Procédé selon l'une des revendications 1, 4 ou 5, caractérisé en ce qu'il est appliqué au chloro-4 phénol, au dichloro-2,4 phénol, aux mélanges de deux d'entre-eux ou aux mélanges de l'un ou de deux d'entre-eux avec d'autres chlorophénols.

**7.** Procédé selon l'une des revendications 1, 4 ou 5, caractérisé en ce qu'il est appliqué au gaïacol, au méthoxy-4 phénol, à l'éthoxy-2 phénol, à l'éthoxy-4 phénol, au pyrocatéchol, à l'hydroxy-2 benzaldéhyde ou à l'hydroxy-4 benzaldéhyde.

**8.** Procédé selon l'une des revendications 1 à 7, caractérisé en ce que le solvant aprotique apolaire est choisi parmi les hydrocarbures aliphatiques, les hydrocarbures aliphatiques chlorés, les chlorobenzènes et les bromobenzènes.

**9.** Procédé selon l'une des revendications 1 à 8, caractérisé en ce que le solvant aprotique apolaire est un hydrocarbure perchloré choisi parmi les alcanes perchlorés ayant 1 à 4 atomes de carbone, les alcènes perchlorés ayant 2 à 4 atomes de carbone et les alcadiènes perchlorés ayant 3 à 6 atomes de carbone.

**10.** Procédé selon la revendication 9, caractérisé en ce que l'hydrocarbure perchloré est le tétrachlorure de carbone ou le tétrachloroéthylène.

**11.** Procédé selon l'une des revendications 1 et 4 à 10, caractérisé en ce que l'on utilise de 0,0010 % à

0,1000 % en poids d'amine ramifiée de formule (II) ou (III) par rapport au solvant aprotique apolaire et de préférence de 0,0050 % à 0,0200 % en poids.

**12.** Procédé selon l'une des revendications 3 à 11, caractérisé en ce que l'amine ramifiée de formule (III) est choisie parmi la diisopropylamine, la tertiobutylamine, la méthyl-1 cyclopentylamine et la méthyl-1 cyclohexylamine.

**13.** Procédé selon l'une des revendications 1 à 12, caractérisé en ce que l'on opère entre 40°C et la température de reflux du milieu réactionnel et de préférence entre 60°C et 120°C.

**14.** Procédé selon l'une des revendications 1 à 13, caractérisé en ce que la concentration initiale en composé phénolique de formule (I), en chloro-2 phénol,ou en mélanges de composés phénoliques est de 1 % à 50 % en poids par rapport au solvant aprotique apolaire et de préférence de 2 % à 10 % en poids.

**15.** Procédé selon l'une des revendications 1 à 14, caractérisé en ce que le rapport molaire chlore/composé phénolique de formule (I) ou chloro-2 phénol est de 0,9 à 1,1.

**16.** Procédé de purification du dichloro-2,6 phénol en mélange avec du dichloro-2,4 phénol, caractérisé en ce que l'on chlore sélectivement le dichloro-2,4 phénol du mélange par le procédé selon l'une des revendications 1 et 4 à 15 et que l'on sépare ensuite de manière connue en soi le dichloro-2,6 phénol du mélange final.

**17.** Procédé de purification du chloro-2 phénol en mélange avec du chloro-4 phénol, caractérisé en ce que l'on chlore sélectivement le chloro-4 phénol du mélange par le procédé selon l'une des revendications 1 et 4 à 15 et que l'on sépare ensuite de manière connue en soi le chloro-2 phénol du mélange final.

**Claims**

**1.** A process for the selective chlorination in the ortho position with respect to the OH function of the phenolic compounds of the general formula (I): wherein:

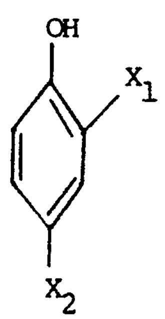

(I)

- $X_1$ represents a chlorine atom, an OH group, an alkyl group having from 1 to 4 carbon atoms, an alkoxy group having from 1 to 4 carbon atoms, an aldehyde group or a hydrogen atom,
- $X_2$ represents a chlorine atom, an OH group, an alkoxy group having from 1 to 4 carbon atoms, an aldehyde group or a hydrogen atom,
- the symbols $X_1$ and $X_2$ do not simultaneously represent a hydrogen atom, and
- the symbol $X_1$ not representing a chlorine atom $X_2$ represents a hydrogen atom,

by means of gaseous chlorine, characterised in that operation is effected in an aprotic apolar solvent and in the presence of an effective amount of an amine of general formula (II):

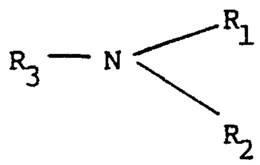

(II)

wherein:

- $R_1$, $R_2$ and $R_3$ which are identical or different represent a straight alkyl radical having from 1 to 10 carbon atoms, a secondary alkyl radical having from 3 to 10 carbon atoms or a tertiary alkyl radical having from 4 to 10 carbon atoms,
- $R_1$, $R_2$ or $R_3$ represents a phenyl radical, a cyclohexyl radical, a cycloheptyl radical or a cyclopentyl radical, and
- one or two of the symbols $R_1$, $R_2$ and $R_3$ may represent a hydrogen atom.

**2.** A process for the selective chlorination in the ortho position with respect to the OH function of 2-chlorophenol which is free from phenol, by means of gaseous chlorine, characterised in that operation is effected in an aprotic apolar solvent and in the presence of from 0.0010% to 0.0200% by weight of an amine of general formula (II), with respect to the aprotic apolar solvent:

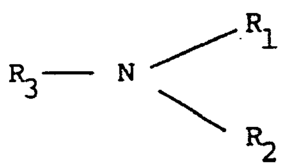

wherein:

- $R_1$, $R_2$ and $R_3$ which are identical or different represent a straight alkyl radical having from 1 to 10 carbon atoms, a secondary alkyl radical having from 3 to 10 carbon atoms or a tertiary alkyl radical having from 4 to 10 carbon atoms,
- $R_1$, $R_2$ or $R_3$ represents a phenyl radical, a cyclohexyl radical, a cycloheptyl radical or a cyclopentyl radical, and
- one or two of the symbols $R_1$, $R_2$ and $R_3$ may represent a hydrogen atom.

**3.** A process according to claim 2 characterised by using a branched chain amine of general formula (III):

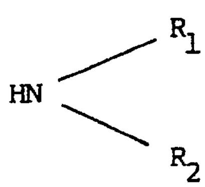

(III)

wherein:

- $R_1$ represents a secondary alkyl radical having from 3 to 10 carbon atoms or a tertiary alkyl radical having from 4 to 10 carbon atoms, and
- $R_2$ represents:
  + a hydrogen atom when $R_1$ is a tertiary alkyl radical, and
  + a secondary alkyl radical having from 3 to 10 carbon atoms when $R_1$ is also a secondary alkyl radical.

**4.** A process according to claim 1 characterised by using a branched chain amine of general formula (II):

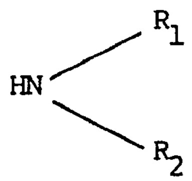

(III)

wherein:

- $R_1$ represents a secondary alkyl radical having from 3 to 10 carbon atoms or a tertiary alkyl radical having from 4 to 10 carbon atoms, and
- $R_2$ represents:
  + a hydrogen atom when $R_1$ is a tertiary alkyl radical, and
  + a secondary alkyl radical having from 3 to 10 carbon atoms when $R_1$ is also a secondary alkyl radical.

**5.** A process according to either one of claims 1 and 4 characterised in that it is applied to phenolic compounds of formula (I) wherein:

- $X_1$ and $X_2$ which are identical or different represent a chlorine atom, an OH group, a methoxy group, an ethoxy group or an aldehyde group,
- only one of which symbols $X_1$ or $X_2$ may represent a hydrogen atom.

**6.** A process according to one of claims 1, 4 and 5 characterised in that it is applied to 4-chlorophenol, 2,4-dichlorophenol, mixtures of two thereof or mixtures of one or two thereof with other chlorophenols.

**7.** A process according to one of claims 1, 4 and 5 characterised in that it is applied to guaiacol, 4-methoxy phenol, 2-ethoxy phenol, 4-ethoxy phenol, pyrocatechol, 2-hydroxy benzaldehyde or 4-hydroxy benzaldehyde.

**8.** A process according to one of claims 1 to 7 characterised in that the aprotic apolar solvent is selected from aliphatic hydrocarbons, chlorinated aliphatic hydrocarbons, chlorobenzenes and bromobenzenes.

**9.** A process according to one of claims 1 to 8 characterised in that the aprotic apolar solvent is a perchlorinated hydrocarbon selected from perchlorinated alkanes having from 1 to 4 carbon atoms, perchlorinated alkenes having from 2 to 4 carbon atoms and perchlorinated alkadienes having from 3 to 6 carbon atoms.

**10.** A process according to claim 9 characterised in that the perchlorinated hydrocarbon is carbon tetrachloride or tetrachloroethylene.

**11.** A process according to one of claims 1 and 4 to 10 characterised by using from 0.0010% to 0.1000% by weight of branched amine of formula (II) or (III) with respect to the aprotic apolar solvent and preferably from 0.0050% to 0.0200% by weight.

**12.** A process according to one of claims 3 to 11 characterised in that the branched amine of formula (III) is selected from diisopropylamine, tertiobutylamine, 1-methyl cyclopentylamine and 1-methyl cyclohexylamine.

**13.** A process according to one of claims 1 to 12 characterised by operating at between 40 °C and the reflux temperature of the reaction medium and preferably between 60 °C and 120 °C.

**14.** A process according to one of claims 1 to 13 characterised in that the initial concentration in respect of phenolic compound of formula (I), 2-chlorophenol or mixtures of phenolic compounds is from 1% to 50% by weight with respect to the aprotic apolar solvent and preferably from 2% to 10% by weight.

**15.** A process according to one of claims 1 to 14 characterised in that the chlorine/phenolic compound of formula (I) or 2-chlorophenol molar ratio is from 0.9 to 1.1.

**16.** A process for the purification of 2,6-dichlorophenol mixed with 2,4-dichlorophenol characterised by

selectively chlorinating the 2,4-dichlorophenol of the mixture by the process according to one of claims 1 and 4 to 15, and then separating the 2,6-dichlorophenol from the final mixture in per se known manner.

**17.** A process for the purification of 2-chlorophenol mixed with 4-chlorophenol characterised by selectively chlorinating the 4-chlorophenol of the mixture by the process according to one of claims 1 and 4 to 15 and then separating the 2-chlorophenol from the final mixture in per se known manner.

**Patentansprüche**

**1.** Verfahren zur selektiven Chlorierung von Phenolverbindungen der allgemeinen Formel (I) in ortho-Stellung, bezogen auf die OH-Gruppe,

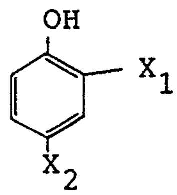

(I)

wobei
- $X_1$ ein Chloratom, eine OH-, eine 1 bis 4 Kohlenstoffatome enthaltende Alkyl-, eine 1 bis 4 Kohlenstoffatome enthaltende Alkoxygruppe, eine Aldehydgruppe oder ein Wasserstoffatom,
- $X_2$ ein Chloratom, eine OH-, eine 1 bis 4 Kohlenstoffatome enthaltende Alkoxygruppe, eine Aldehydgruppe oder ein Wasserstoffatom bedeutet,
- $X_1$ und $X_2$ gleichzeitig keine Wasserstoffatome sein dürfen und
- $X_1$ kein Chloratom ist, wenn $X_2$ ein Wasserstoffatom bedeutet,

mit Chlorgas, dadurch gekennzeichnet, daß man in einem apolaren aprotischen Lösungsmittel und in Gegenwart einer wirksamen Menge eines Amins der allgemeinen Formel (II) arbeitet,

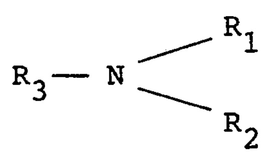

(II)

in der
- $R_1$, $R_2$ und $R_3$ gleich oder verschieden sind und einen geradkettigen 1 bis 10 Kohlenstoffatome enthaltenden Alkylrest, einen 3 bis 10 Kohlenstoffatome enthaltenden sekundären Alkylrest oder einen 4 bis 10 Kohlenstoffatome enthaltenden tertiären Alkylrest bedeuten,
- $R_1$, $R_2$ oder $R_3$ einen Phenyl-, Cyclohexyl-, Cycloheptyl- oder Cyclopentylrest bedeuten und
- eines oder zwei der Symbole $R_1$, $R_2$ und $R_3$ ein Wasserstoffatom bedeuten können.

**2.** Verfahren zur selektiven Chlorierung von phenolfreiem 2-Chlorphenol in ortho-Stellung, bezogen auf die OH-Gruppe, mit Chlorgas, dadurch gekennzeichnet, daß man in einem apolaren aprotischen Lösungsmittel und in Gegenwart von 0,0010 bis 0,0200 Gew.%, bezogen auf das apolare aprotische Lösungsmittel, eines Amins der allgemeinen Formel (II) arbeitet,

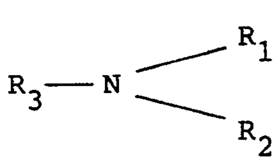

(II)

in der
- $R_1$, $R_2$ und $R_3$ gleich oder verschieden sind und einen 1 bis 10 Kohlenstoffatome enthaltenden

geradkettigen Alkyl-, einen 3 bis 10 Kohlenstoffatome enthaltenden sekundären Alkyl- oder einen 4 bis 10 Kohlenstoffatome enthaltenden tertiären Alkylrest bedeuten,
- $R_1$, $R_2$ oder $R_3$ Phenyl-, Cyclohexyl-, Cycloheptyl- oder Cyclopentylrest bedeuten und
- einer oder zwei der Reste $R_1$, $R_2$ und $R_3$ ein Wasserstoffatom bedeuten können.

**3.** Verfahren nach Anspruch 2, dadurch gekennzeichnet, daß man ein verzweigtkettiges Amin der allgemeinen Formel (III) verwendet,

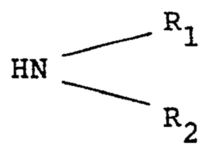

(III)

in der
- $R_1$ einen 3 bis 10 Kohlenstoffatome enthaltenden sekundären Alkylrest oder einen 4 bis 10 Kohlenstoffatome enthaltenden tertiären Alkylrest und
- $R_2$
+ ein Wasserstoffatom bedeutet, wenn $R_1$ ein tertiärer Alkylrest und
+ einen 3 bis 10 Kohlenstoffatome enthaltenden sekundären Alkylrest bedeutet, wenn $R_1$ ebenfalls ein sekundärer Alkylrest ist.

**4.** Verfahren nach Anspruch 1, dadurch gekennzeichnet, daß man ein verzweigtkettiges Amin der allgemeinen Formel (III) verwendet,

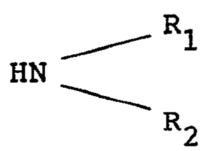

(III)

in der
- $R_1$ einen 3 bis 10 Kohlenstoffatome enthaltenden sekundären Alkylrest oder einen 4 bis 10 Kohlenstoffatome enthaltenden tertiären Alkylrest und
- $R_2$
+ ein Wasserstoffatom bedeutet, wenn $R_1$ ein tertiärer Alkylrest und
+ einen 3 bis 10 Kohlenstoffatome enthaltenden sekundären Alkylrest bedeutet, wenn $R_1$ ebenfalls ein sekundärer Alkylrest ist.

**5.** Verfahren nach Anspruch 1 oder 4, dadurch gekennzeichnet, daß es auf Phenolverbindungen der Formel (I) angewendet wird, in der
- $X_1$ und $X_2$ gleich oder verschieden sind und ein Chloratom, eine OH-, Methoxy-, Ethoxy- oder Aldehydgruppe bedeuten und
- nur eines der Symbole $X_1$ oder $X_2$ ein Wasserstoffatom bedeuten kann.

**6.** Verfahren nach einem der Ansprüche 1, 4 oder 5, dadurch gekennzeichnet, daß es auf 4-Chlorphenol, 2,4-Dichlorphenol, Gemische von beiden oder auf Gemische von einem oder beiden mit anderen Chlorphenolen angewendet wird.

**7.** Verfahren nach einem der Ansprüche 1, 4 oder 5, dadurch gekennzeichnet, daß es auf Guajakol, 4-Methoxyphenol, 2-Ethoxyphenol, 4-Ethoxyphenol, Brenzkatechin, 2-Hydroxybenzaldehyd oder 4-Hydroxybenzaldehyd angewendet wird.

**8.** Verfahren nach einem der Ansprüche 1 bis 7, dadurch gekennzeichnet, daß das apolare aprotische Lösungsmittel aus den aliphatischen Kohlenwasserstoffen und chlorierten aliphatischen Kohlenwasserstoffen, den Chlorbenzolen und Brombenzolen ausgewählt wird.

9. Verfahren nach einem der Ansprüche 1 bis 8, dadurch gekennzeichnet, daß das apolare aprotische Lösungsmittel ein perchlorierter Kohlenwasserstoff ist, der aus perchlorierten 1 bis 4 Kohlenstoffatome enthaltenden Alkanen, perchlorierten 2 bis 4 Kohlenstoffatome enthaltenden Alkenen und perchlorierten 3 bis 6 Kohlenstoffatome enthaltenden Alkadienen ausgewählt ist.

10. Verfahren nach Anspruch 9, dadurch gekennzeichnet, daß der perchlorierte Kohlenwasserstoff Tetrachlorkohlenstoff oder Tetrachlorethylen ist.

11. Verfahren nach einem der Ansprüche 1 und 4 bis 10, dadurch gekennzeichnet, daß man 0,0010 bis 0,1000 Gew.% und vorzugsweise 0,0050 bis 0,0200 Gew.%, bezogen auf das apolare aprotische Lösungsmittel, verzweigtes Amin der Formel (II) oder (III) verwendet.

12. Verfahren nach einem der Ansprüche 3 bis 11, dadurch gekennzeichnet, daß das verzweigte Amin der Formel (III) aus Diisopropylamin, tert.-Butylamin, 1-Methylcyclopentylamin und 1-Methylcyclohexylamin ausgewählt ist.

13. Verfahren nach einem der Ansprüche 1 bis 12, dadurch gekennzeichnet, daß man zwischen 40 °C und der Rückflußtemperatur des Reaktionsmilieus und vorzugsweise zwischen 60 und 120 °C arbeitet.

14. Verfahren nach einem der Ansprüche 1 bis 13, dadurch gekennzeichnet, daß die Anfangskonzentration der Phenolverbindung der Formel (I), des 2-Chlorphenols oder der Gemische aus Phenolverbindungen 1 bis 50 Gew.% und vorzugsweise 2 bis 10 Gew.%, bezogen auf das apolare aprotische Lösungsmittel, beträgt.

15. Verfahren nach einem der Ansprüche 1 bis 14, dadurch gekennzeichnet, daß das Molverhältnis Chlor/Phenolverbindung der Formel (I) oder 2-Chlorphenol 0,9 bis 1,1 beträgt.

16. Verfahren zur Reinigung von mit 2,4-Dichlorphenol gemischtem 2,6-Dichlorphenol, dadurch gekennzeichnet, daß man das im Gemisch vorliegende 2,4-Dichlorphenol selektiv durch das Verfahren nach einem der Ansprüche 1 und 4 bis 15 chloriert und daß man anschließend auf an sich bekannte Weise das 2,6-Dichlorphenol vom Endgemisch abtrennt.

17. Verfahren zur Reinigung von mit 4-Chlorphenol gemischtem 2-Chlorphenol, dadurch gekennzeichnet, daß man das im Gemisch vorliegende 4-Chlorphenol selektiv durch das Verfahren nach einem der Ansprüche 1 und 4 bis 15 chloriert und daß man anschließend auf an sich bekannte Weise das 2-Chlorphenol vom Endgemisch abtrennt.